# EUROPEAN PATENT APPLICATION

(11) **EP 2 944 344 A1**
(43) Date of publication of application: **18.11.2015**
(21) Application number: 15162959.9
(22) Date of filing: 09.04.2015
(51) Int. Cl.: A61M 25/00, A61M 25/01

(54) **CATHETER**

(30) Priority: 13.05.2014 JP 2014099339
(71) Applicant: ASAHI INTECC CO., LTD., Nagoya-shi, Aichi 463-0024 (JP)
(72) Inventor: NISHIGISHI, Makoto c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(57) **Abstract**

[Problem to be Solved]

An object of the present invention is to provide a catheter that does not reduce the thickness of an inner tube in a welded part and reduces the risk of penetration of a guide wire through the welded part.

[Solution]

In catheters 10, 10a, and 10b and a balloon catheter 10c, a protective film 40, 40a, or 40b made of a resin having higher heat resistance than a first inner tube 50 is interposed between an outer tube 30 and the first inner tube 50. Even if the first inner tube 50 is made of a soft thermoplastic resin, this configuration does not reduce the thickness of the first inner tube 50 in the welded part 100 and can reduce the risk of penetration of the guide wire, which is inserted into the first inner tube 50 by an operator, through the first welded part 110, 110a, 110b, or 110c to the walls of a blood vessel, a bile duct, a pancreatic duct, and so on from the first inner tube 50 near the outer tube 30.

## Description

### [Technical Field]

The present invention relates to a catheter that improves selection of a blood vessel for a guide wire at a branch point.

### [Background Art]

The formation of a stenosis or occlusion site in a blood vessel, a bile duct, a pancreatic duct, and so on may deteriorate flows of blood, bile (gall), pancreatic juice, and so on. Conventionally, in a widely used treatment, a guide wire is inserted into a stenosis or occlusion site, and then the stenosis or the occlusion site is extended by a balloon catheter that travels along a guide wire.

Generally, the number of branch points between a thick vessel (main branch) and thin vessels (side branches) increases toward the end of a blood vessel, a bile duct, or a pancreatic duct. Thus, for a guide wire, blood vessels need to be selected to change a traveling direction at a branch point from a thick vessel (main branch) to a thin vessel (side branch). However, the insertion of the guide wire to the end of a blood vessel, a bile duct, or a pancreatic duct reduces transmission of a pressing force or a turning force applied by an operator to the end of the guide wire. Thus, in the event of a stenosis or occlusion site on the end of a blood vessel, a bile duct, or a pancreatic duct, it is difficult to change a traveling direction at a branch point from a thick vessel (main branch) to a thin vessel (side branch) with the guide wire alone.

Hence, as a catheter for improving the selection of blood vessels for a guide wire, a known dual lumen catheter includes a first inner tube and a second inner tube that are inserted into an outer tube (For example, see Patent Literatures 1 and 2 described below).

In the catheter of Patent Literature 1, a first guide wire is inserted into the first inner tube made of a thermoplastic resin; meanwhile, a second guide wire is inserted into the second inner tube made of a thermoplastic resin. Thus, the backup property of the second guide wire is improved by the first guide wire, leading to improved selection of blood vessels for the second guide wire. In the catheter of Patent Literature 2, the first inner tube composed of a metal coil improves the operability of the catheter.

In the catheter of Patent Literature 1, however, the outer tube, the first inner tube, and the second inner tube are welded with a heat shrinkable tube. At this point, these tubes are all made of a thermoplastic resin and thus are melted together in a welded part. The heat shrinkable tube presses molten resin out of the welded part so as to reduce a tube thickness (the thickness of the outer tube, the thickness of the first inner tube, and the thickness of the second inner tube) in the welded part. Thus, when an operator inserts the first guide wire into the first inner tube, the end of the first guide wire may easily penetrate the welded part where the tube has a small thickness. Unfortunately, this may cause the first guide wire at the welded part to penetrate the walls of a blood vessel, a bile duct, a pancreatic duct, and so on from the outer tube side of the first inner tube or may insert the first guide wire at the welded part into the second inner tube from the second inner tube side of the first inner tube, precluding insertion of the second guide wire into the second inner tube. In the catheter of Patent Literature 2, the first inner tube composed of a metal coil increases a frictional resistance between the first inner tube and the first guide wire, leading to difficulty in inserting the first guide wire into the first inner tube.

### [Citation List]

### [Patent Literature]

[Patent Literature 1] WO 2006/126642
[Patent Literature 2] U.S. Patent No. 8657845

### [Summary of Invention]

### [Technical Problem]

The present invention has been devised in view of these circumstances. An obj ect of the present invention is to provide a catheter including an outer tube and an inner tube made of a thermoplastic resin, that is inserted into the outer tube and is welded to the outer tube in a welded part, having a reduced risk of penetration of a guide wire through the welded part.

### [Solution to Problem]

The problem can be solved by a catheter according to any one of the claims. Preferred aspects of the catheter, being subject-matter of dependent claims, are discussed in the following in more details:

A first aspect of the present invention is a catheter including an outer tube; a first inner tube that is inserted into the outer tube and is welded to the outer tube in a first welded part; and a protective film that is interposed between the outer tube and the first inner tube and is provided at least in a part of the first welded part, the first inner tube being made of a thermoplastic resin, the protective film being made of a resin having higher heat resistance than the resin of the first inner tube.

A second aspect of the present invention is a catheter according to the first aspect, further including a second inner tube that is inserted into the outer tube and is welded to the first inner tube in a second welded part, wherein a protective film having higher heat resistance than the resin of the first inner tube is interposed between the first inner tube and the second inner tube and is provided at least in a part of the second welded part. The protective film interposed between the first inner tube and the second inner tube may be formed by the protective film interposed between the first inner tube and the outer tube, or can be a protective film made of resin provided separately from the protective film interposed between the first inner tube and the outer tube. In the latter case, the two protective films can be made of a same resin material.

A third aspect of the present invention is a catheter according to the first or second aspect, wherein the protective film interposed between the first inner tube and the outer tube and/or the protective film interposed between the first inner tube and the second inner tube have/has an axial length that is equal to or longer than the axial length of the first welded part and/or the second welded part.

A fourth independent aspect of the present invention is a balloon catheter including the catheter according to any one of the above described aspects, and a balloon welded to the front end of the outer tube.

### [Advantageous Effects of Invention]

In the catheter according to the first aspect of the present invention, the protective film made of a resin having higher heat resistance than the resin of the first inner tube is held at least in a part of the first welded part where the outer tube and the first inner tube are welded to each other. Thus, even if the first inner tube is made of a soft thermoplastic resin, the protective film made of a resin having high heat resistance reduces the risk of excessive melting of the first inner tube near the outer tube during welding, leading to smaller risk of reducing the thickness of the first inner tube near the outer tube in the first welded part. Thus, when the guide wire is inserted into the first inner tube by an operator, this configuration can reduce the risk of penetration of the guide wire through the first welded part to the walls of a blood vessel, a bile duct, a pancreatic duct, and so on from the first inner tube near the outer tube.

In the catheter according to the second aspect of the present invention, the protective film is held at least in a part of the second welded part where the first inner tube and the second inner tube are welded to each other. Thus, the protective film made of a resin having high heat resistance reduces the risk of excessive melting of the first inner tube near the second inner tube during welding, leading to smaller risk of reducing the thickness of the first inner tube near the second inner tube in the second welded part. Thus, when the guide wire is inserted into the first inner tube by the operator, this configuration can reduce the risk of penetration of the guide wire through the second welded part into the second inner tube from the first inner tube near the second inner tube.

In the catheter according to the third aspect of the present invention, the protective film has an axial length that is equal to or longer than the axial length of the first welded part and the second welded part. This configuration reduces both the risk of directly welding the outer tube and the first inner tube in the first welded part and the risk of directly welding the first inner tube and the second inner tube in the second welded part. Moreover, this configuration can reduce the risk of reducing the thickness of the first inner tube near the outer tube and the thickness of the first inner tube near the second inner tube along the lengths of the first welded part and the second welded part. Thus, when the guide wire is inserted into the first inner tube by the operator, this configuration can reduce the risk of penetration of the guide wire through the first welded part to the walls of a blood vessel, a bile duct, a pancreatic duct, and so on from the first inner tube near the outer tube and the risk of penetration of the guide wire through the second welded part into the second inner tube from the first inner tube near the second inner tube.

The balloon catheter according to the fourth aspect of the present invention includes the catheter according to any one of the first to third aspects, and a balloon welded to the front end of the outer tube. Thus, the balloon is inflated to the walls of a blood vessel, a bile duct, a pancreatic duct, and so on to fix the front end of the balloon catheter, thereby further improving the backup property of the second guide wire. This can further improve the selection of a blood vessel for the second guide wire.

### [Brief Description of Drawings]

[FIG. 1]

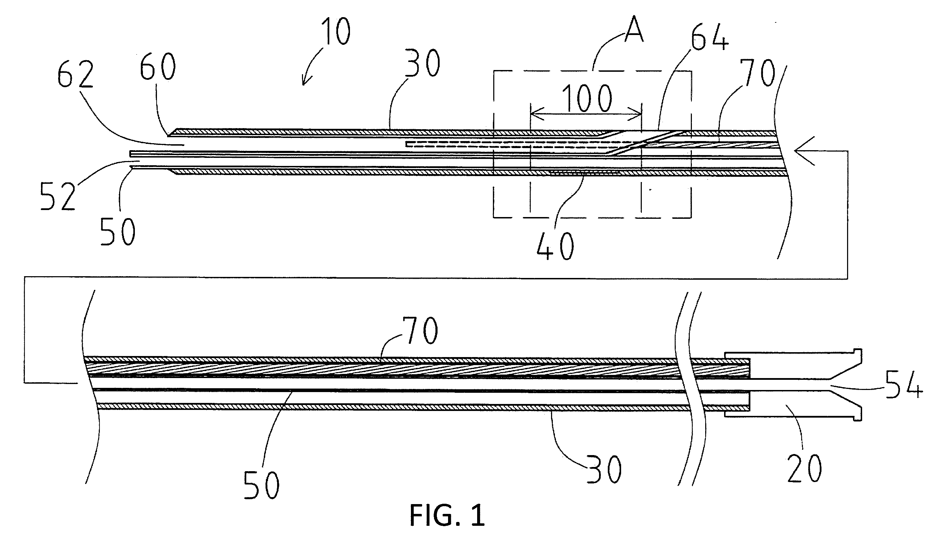

   FIG. 1 is an overall view of a catheter according to the present embodiment.
[FIG. 2]

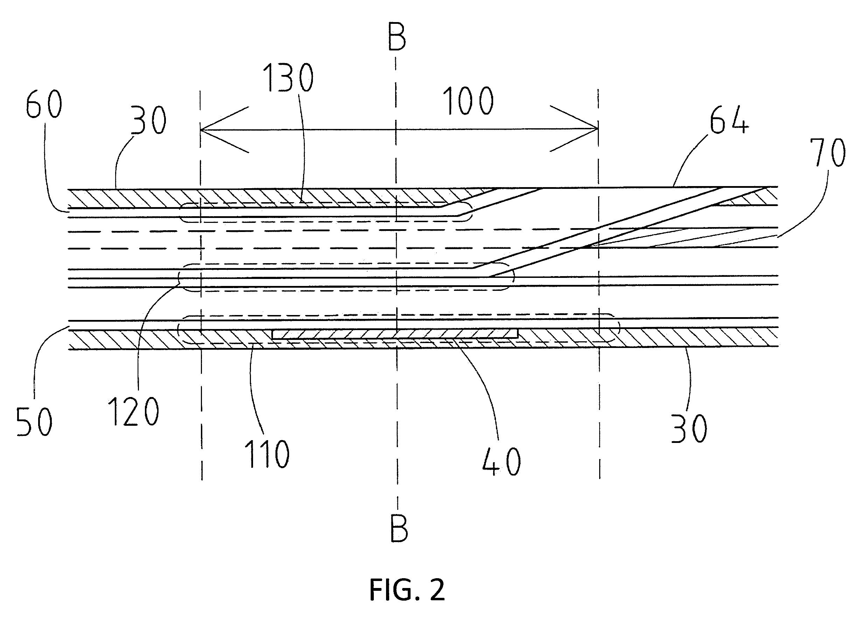

   FIG. 2 is an enlarged view of part A of FIG. 1.
[FIG. 3]

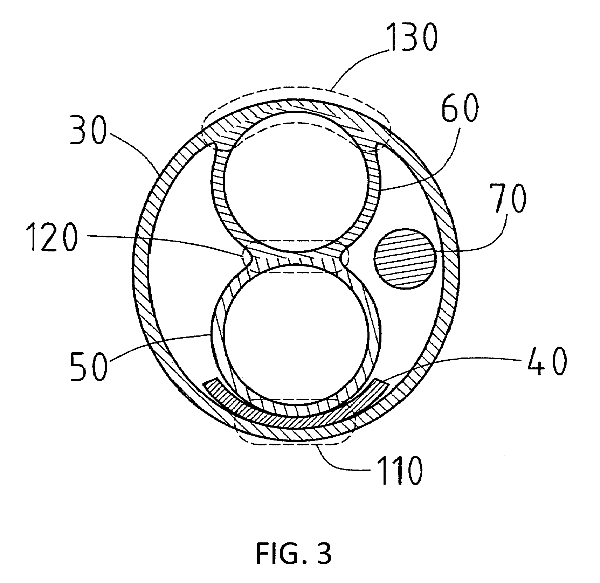

   FIG. 3 is a cross-sectional view taken along line B-B of FIG. 2.
[FIG. 4]

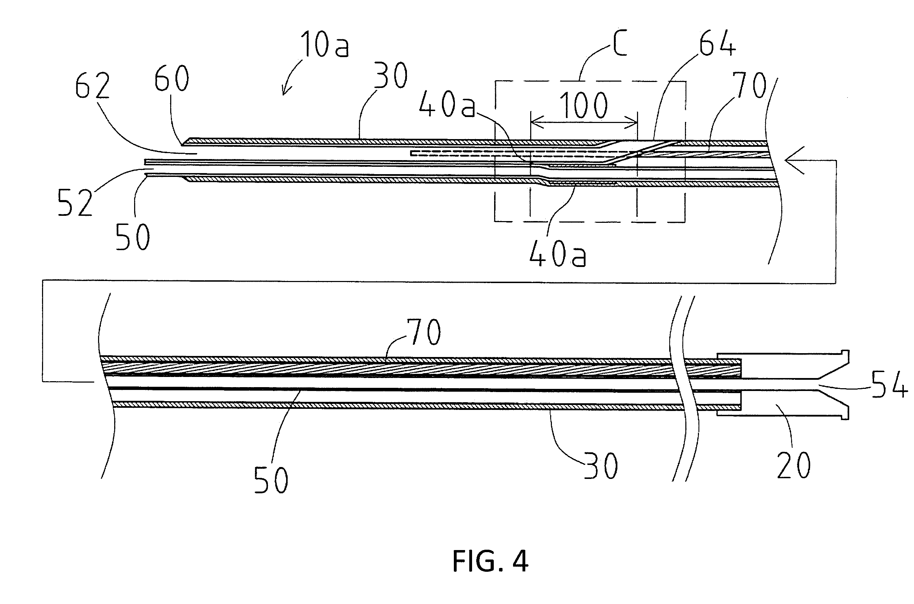

   FIG. 4 is an overall view of a catheter according to a second embodiment.
[FIG. 5]

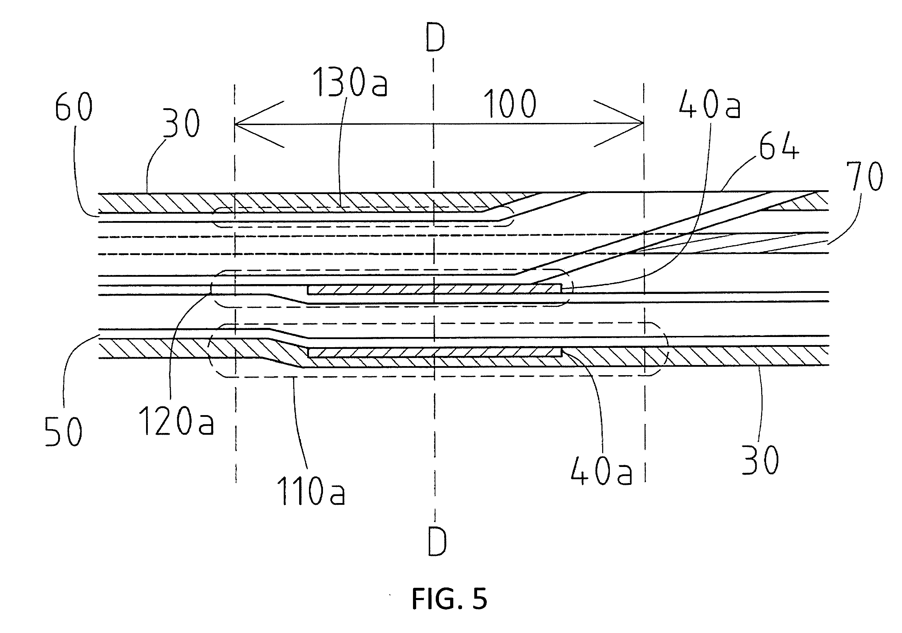

   FIG. 5 is an enlarged view of part C of FIG. 4.
[FIG. 6]

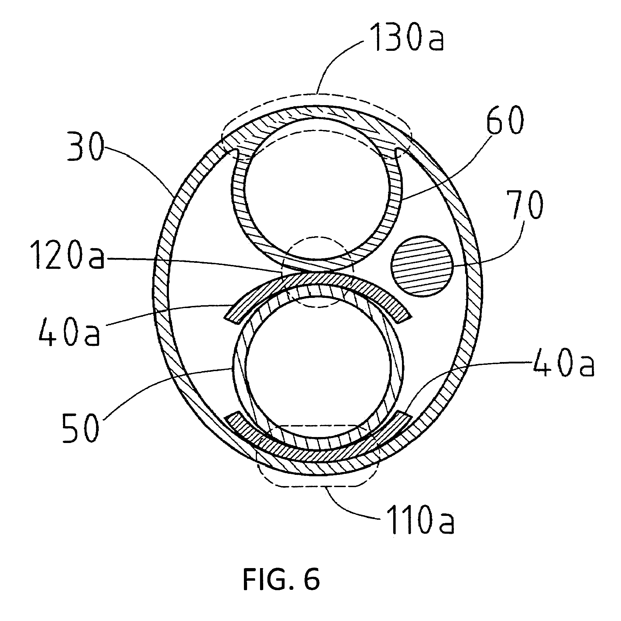

   FIG. 6 is a cross-sectional view taken along line D-D of FIG. 5.
[FIG. 7]

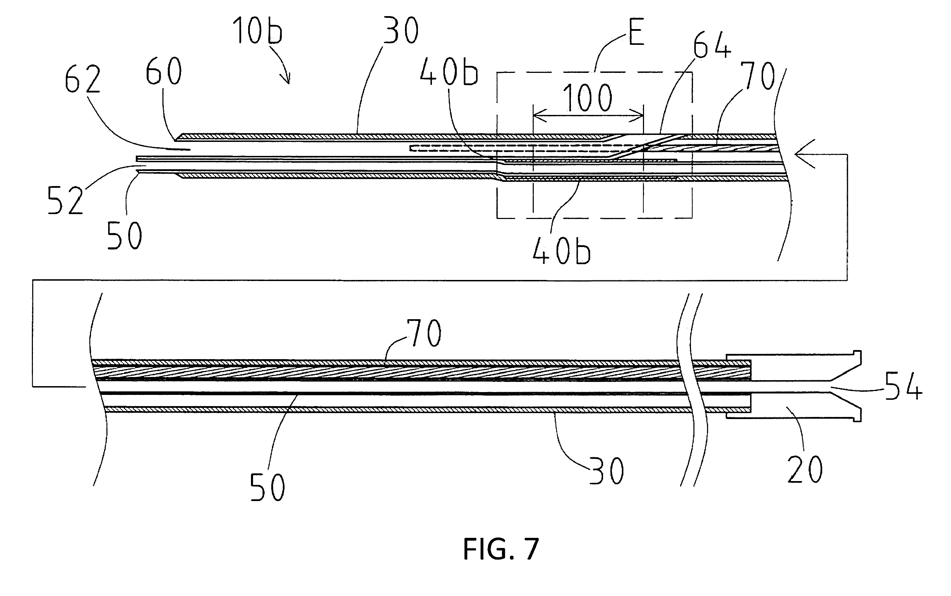

   FIG. 7 is an overall view of a catheter according to a third embodiment.
[FIG. 8]

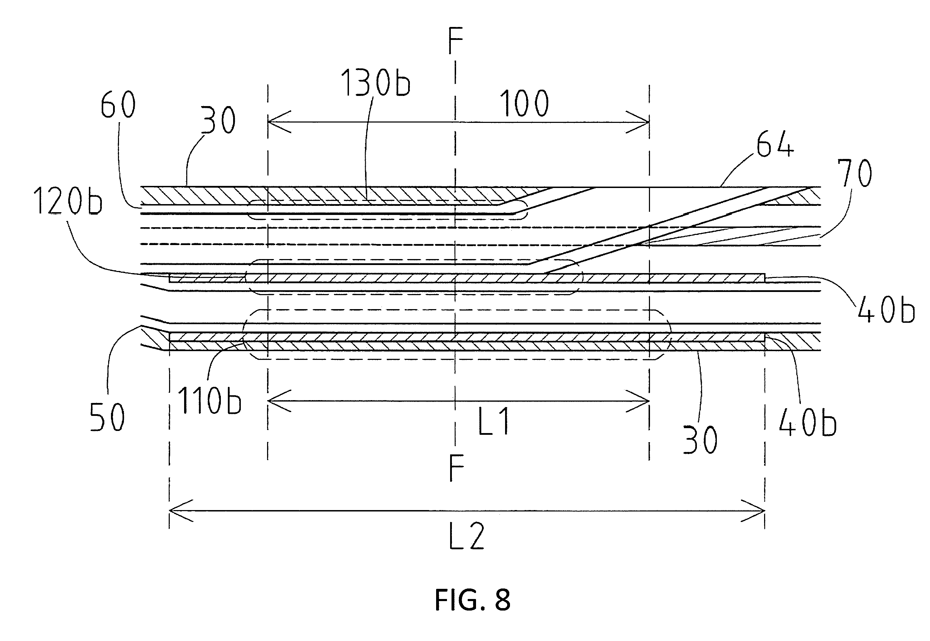

   FIG. 8 is an enlarged view of part E of FIG. 7.
[FIG. 9]

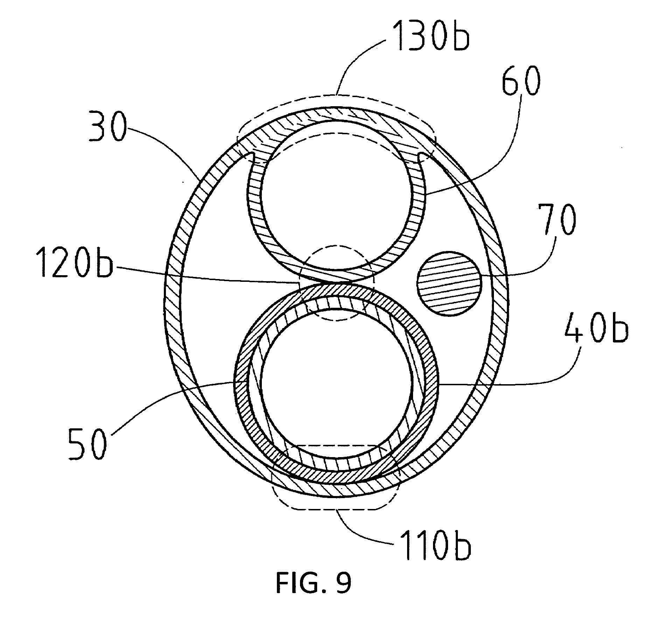

   FIG. 9 is a cross-sectional view taken along line F-F of FIG. 8.
[FIG. 10]

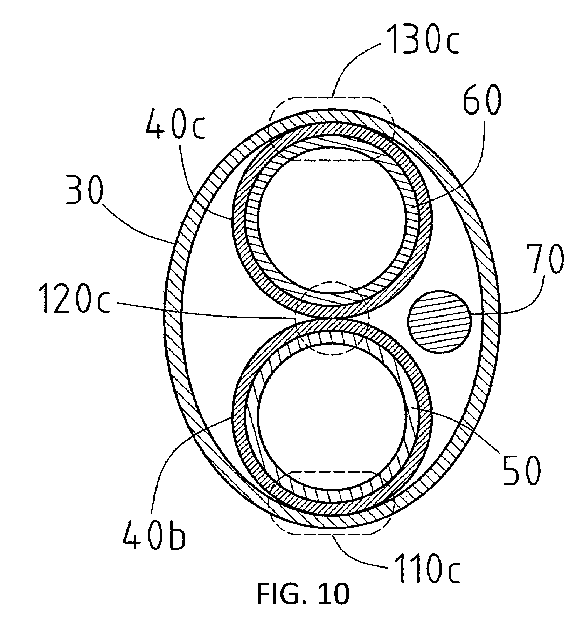

   FIG. 10 shows a modification of FIG. 9.
[FIG. 11]

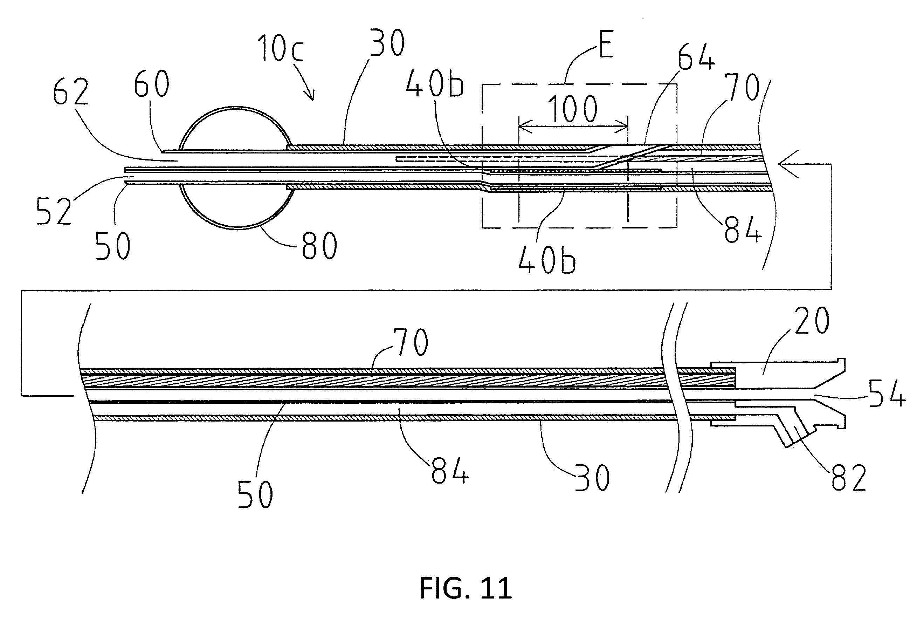

   FIG. 11 is an overall view of a catheter according to a fourth embodiment.

### [Description of Embodiments]

Referring to FIGS. 1 to 3, a catheter 10 according to the present embodiment is used in the following example. In FIGS. 1 and 2, the left side indicates a front end (distal side) to be inserted into a body while the right side indicates a rear end (proximal side) operated by an operator, e.g., a doctor.

For example, the catheter 10 is used for improving selection of a blood vessel for a guide wire at a branch point. As shown in FIG. 1, the catheter 10 mainly includes a connector 20, an outer tube 30, a first inner tube 50, a second inner tube 60, and a reinforcing member 70.

In the outer tube 30, the first inner tube 50 is inserted along the length of the catheter 10. The guide wire can be inserted into the first inner tube 50. In order to facilitate the insertion of the guide wire, the connector 20 is connected to the rear end of the outer tube 30 and the rear end of the first inner tube 50. The front end of the first inner tube 50 has a first front-end opening 52 while a first insertion opening 54 is provided on the rear end of the first inner tube 50 via the connector 20.

In the outer tube 30, the second inner tube 60 is inserted from a midpoint to the front end of the catheter 10 parallel with the first inner tube 50. As in the first inner tube 50, the guide wire can be inserted into the second inner tube 60. The front end of the second inner tube 60 has a second front-end opening 62 while the rear end of the second inner tube 60 has a second insertion opening 64.

The outer tube 30, the first inner tube 50, and the second inner tube 60 may be made of thermoplastic resins such as polyamide, polyamide elastomer, polyolefin, polyester, polyester elastomer, and nylon.

Since the first inner tube 50 is extended along the length of the catheter 10, the length of the first inner tube 50 may disadvantageously lead to difficulty in replacement of the guide wire to be inserted by an operator. However, the insertion of the guide wire into the first inner tube 50 may improve the stiffness of the catheter 10, advantageously allowing an operator to easily press the catheter 10 in the distal direction. Moreover, the second inner tube 60 is only extended from a midpoint to the front end of the catheter 10 and thus allows the operator to easily change the guide wire inserted into the second inner tube 60. However, even if the guide wire is inserted into the second inner tube 60, only the front end of the catheter 10 increases in stiffness, disadvantageously causing a break on the catheter 10 around the second insertion opening 64 of the second inner tube 60 that rapidly fluctuates in stiffness when the operator presses the catheter 10 in the distal direction. Since the catheter 10 includes the first inner tube 50 and the second inner tube 60, the operator can quickly change a second guide wire inserted into the second inner tube 60 while a first guide wire is inserted into the first inner tube 50. Furthermore, the catheter 10 is easily pressed in the distal direction.

FIG. 2 is an enlarged view of part A of FIG. 1. FIG. 3 is a cross-sectional view taken along line B-B of FIG. 2. As shown in FIGS. 2 and 3, the outer tube 30, the first inner tube 50, and the second inner tube 60 are welded in a welded part 100. A protective film 40 is interposed between the outer tube 30 and the first inner tube 50. The protective film 40 is made of a resin having higher heat resistance than the resin of the first inner tube 50. In the present embodiment, the protective film 40 is made of polyimide.

As shown in FIGS. 2 and 3, the welded part 100 includes a first welded part 110 where the outer tube 30, the protective film 40, and the first inner tube 50 are welded to one another, a second welded part 120 where the first inner tube 50 and the second inner tube 60 are welded to each other, and a third welded part 130 where the second inner tube 60 and the outer tube 30 are welded to each other. In the second welded part 120, the first inner tube 50 made of a thermoplastic resin and the second inner tube 60 made of a thermoplastic resin are directly welded to each other and thus are reduced in thickness. Likewise, in the third welded part 130, the second inner tube 60 made of a thermoplastic resin and the outer tube 30 are directly welded to each other and thus are reduced in thickness.

In the first welded part 110, the protective film 40 made of a highly heat resistant resin is interposed between the outer tube 30 and the first inner tube 50. The protective film 40 is provided at least on a part of the first welded part 110. Thus, even if the first inner tube 50 is made of a thermoplastic resin, this configuration reduces the risk of excessive melting of the first inner tube 50 next to the outer tube 30 during welding, leading to smaller risk of reducing the thickness of the first inner tube 50 next to the outer tube 30 in the first welded part 110. Thus, when the guide wire is inserted into the first inner tube 50 by the operator, this configuration can reduce the risk of penetration of the guide wire through the first welded part 110 to the walls of a blood vessel, a bile duct, a pancreatic duct, and so on from the first inner tube 50 near the outer tube 30.

Referring to FIGS. 4 to 6, a catheter 10a according to a second embodiment will be described below. In FIGS. 4 and 5, as in FIGS. 1 and 2, the left side indicates a front end (distal side) to be inserted into a body while the right side indicates a rear end (proximal side) operated by an operator, e.g., a doctor.

Only differences from the catheter 10 of FIGS. 1 to 3 will be described below. In the catheter 10a, a protective film 40a is interposed not only between an outer tube 30 and a first inner tube 50 but also between the first inner tube 50 and a second inner tube 60. As in the catheter 10, the protective film 40a is made of a resin having higher heat resistance than the resin of the first inner tube 50.

FIG. 5 is an enlarged view of part C of FIG. 4. FIG. 6 is a cross-sectional view taken along line D-D of FIG. 5. As shown in FIGS. 5 and 6, a welded part 100 includes a first welded part 110a where the outer tube 30, the protective film 40a, and the first inner tube 50 are welded to one another, a second welded part 120a where the first inner tube 50, the protective film 40a, and the second inner tube 60 are welded to one another, and a third welded part 130a where the second inner tube 60 and the outer tube 30 are welded to each other.

In the second welded part 120a, the protective film 40a made of a highly heat resistant resin is interposed between the first inner tube 50 and the second inner tube 60 as in the first welded part 110. The protective film 40a is provided at least on a part of the second welded part 120a. Thus, even if the first inner tube 50 is made of a thermoplastic resin, this configuration reduces the risk of excessive melting of the first inner tube 50 near the second inner tube 60 during welding, leading to smaller risk of reducing the thickness of the first inner tube 50 near the second inner tube 60 in the second welded part 120a. Thus, when the guide wire is inserted into the first inner tube 50 by the operator, this configuration can reduce the risk of penetration of the guide wire through the second welded part 120a into the second inner tube 60 from the first inner tube 50 near the second inner tube 60.

Referring to FIGS. 7 to 10, a catheter 10b according to a third embodiment will be described below. In FIGS. 7 and 8, as in FIGS. 4 and 5, the left side indicates a front end (distal side) to be inserted into a body while the right side indicates a rear end (proximal side) operated by an operator, e.g., a doctor.

FIG. 8 is an enlarged view of part E of FIG. 7. FIG. 9 is a cross-sectional view taken along line F-F of FIG. 8. Only differences from the catheter 10a of FIGS. 4 to 6 will be described below. As shown in FIGS. 8 and 9, the catheter 10b includes a protective film 40b covering the outer periphery of a first inner tube 50. As in the catheter 10a, the protective film 40b is made of a resin having higher heat resistance than the resin of the first inner tube 50. A welded part 100 includes a first welded part 110b where an outer tube 30, the protective film 40b, and the first inner tube 50 are welded to one another, a second welded part 120b where the first inner tube 50, the protective film 40b, and a second inner tube 60 are welded to one another, and a third welded part 130b where the second inner tube 60 and the outer tube 30 are welded to each other.

As shown in FIG. 8, an axial length L2 of the protective film 40b is longer than an axial length L1 of the welded part 100. This configuration reduces the risk of directly welding the outer tube 30 and the first inner tube 50 in the first welded part 110b and the risk of directly welding the first inner tube 50 and the second inner tube 60 in the second welded part 120b. Moreover, this configuration can reduce the risk of reducing the thickness of the first inner tube 50 near the outer tube 30 and the thickness of the first inner tube 50 near the second inner tube 60 along the lengths of the first welded part 110b and the second welded part 120b (See FIG. 9). Thus, when a guide wire is inserted into the first inner tube 50 by the operator, this configuration can reduce the risk of penetration of the guide wire through the first welded part 110b to the walls of a blood vessel, a bile duct, a pancreatic duct, and so on from the first inner tube 50 near the outer tube 30 and the risk of penetration of the guide wire through the second welded part 120b into the second inner tube 60 from the first inner tube 50 near the second inner tube 60.

In the catheter 10b, the axial length L2 of the protective film 40b is longer than the axial length L1 of the welded part 100. The present invention is not limited to this configuration. For example, even if the axial length L2 of the protective film 40b is equal to the axial length L1 of the welded part 100 (L2 =L1), this configuration reduces the risk of directly welding the outer tube 30 and the first inner tube 50 in the first welded part 110b and the risk of directly welding the first inner tube 50 and the second inner tube 60 in the second welded part 120b.

The protective film 40b is provided only on the outer periphery of the first inner tube 50. The present invention is not limited to this configuration. As shown in FIG. 10, a protective film 40c may be provided on the outer periphery of the second inner tube 60. The protective film 40c is preferably made of a resin having higher heat resistance than the resin of the second inner tube 60. With this configuration, the welded part 100 includes a first welded part 110c where the outer tube 30, the protective film 40b, and the first inner tube 50 are welded to one another, a second welded part 120c where the first inner tube 50, the protective film 40b, the protective film 40c, and the second inner tube 60 are welded to one another, and a third welded part 130c where the second inner tube 60, the protective film 40c, and the outer tube 30 are welded to one another.

In the first welded part 110c, the protective film 40b made of a highly heat resistant resin is interposed between the outer tube 30 and the first inner tube 50. In the second welded part 120c, the protective film 40b and the protective film 40c that are made of a highly heat resistant resin are interposed between the first inner tube 50 and the second inner tube 60. In the third welded part 130c, the protective film 40c made of a highly heat resistant resin is interposed between the second inner tube 60 and the outer tube 30. Even if the first inner tube 50 and the second inner tube 60 are made of a thermoplastic resin, this configuration reduces the risk of excessive melting of the first inner tube 50 and the second inner tube 60 during welding, leading to smaller risk of reducing the thicknesses of the first inner tube 50 and the second inner tube 60 in the welded part 100.

Referring to FIG. 11, a balloon catheter 10c according to a fourth embodiment will be described below. In FIG. 11, as in FIG. 7, the left side indicates a front end (distal side) to be inserted into a body while the right side indicates a rear end (proximal side) operated by an operator, e.g., a doctor.

Only differences from the catheter 10b of FIGS. 7 to 10 will be described below. As shown in FIG. 11, the balloon catheter 10c includes a balloon 80 welded to the front end of an outer tube 30. The front end of the balloon 80 is welded to a first inner tube 50 and a second inner tube 60.

The connector 20 includes a supply port 82 for supplying a liquid such as a contrast medium and a physiological saline. An indeflator (not shown) can be attached to the supply port 82. A liquid supplied from the indeflator passes through an inflation lumen 84 formed by the outer tube 30 and the first inner tube 50 and inflates the balloon 80.

Thus, in the event of a stenosis or occlusion site on the end of a blood vessel, a bile duct, or a pancreatic duct, it is difficult to change a traveling direction at a branch point from a thick vessel (main branch) to a thin vessel (side branch) with the first guide wire alone. In this case, the operator inserts the balloon catheter 10c along the first guide wire. Subsequently, a liquid is supplied from the supply port 82 to inflate the balloon 80 to the walls of a blood vessel, a bile duct, a pancreatic duct, and so on, fixing the front end of the balloon catheter 10c. In this state, the second guide wire is inserted into the second inner tube 60. The front end of the balloon catheter 10c near a branch point is fixed by the balloon 80, thereby remarkably improving the backup property of the second guide wire. This increases the level of selection of a vessel for the second guide wire, facilitating a change of a traveling direction from a thick vessel (main branch) to a thin vessel (side branch).

The balloon catheter 10c includes the balloon 80 welded to the catheter 10b. The present invention is not limited to this configuration. The balloon 80 may be welded to the catheter 10 or 10a to form a balloon catheter. As described above, also in this balloon catheter, the front end of the balloon catheter near a branch point is fixed by the balloon 80 so as to improve the selection of a vessel for the second guide wire. This facilitates a change of a traveling direction from a thick vessel (main branch) to a thin vessel (side branch).

Thus, in the catheters 10, 10a, and 10b and the balloon catheter 10c, the protective film 40, 40a, or 40b made of a resin having higher heat resistance than the first inner tube 50 is interposed between the outer tube 30 and the first inner tube 50. Even if the first inner tube 50 is made of a soft thermoplastic resin, this configuration does not reduce the thickness of the first inner tube 50 in the welded part 100 and can reduce the risk of penetration of the guide wire, which is inserted into the first inner tube 50 by an operator, through the first welded part 110, 110a, 110b, or 110c to the walls of a blood vessel, a bile duct, a pancreatic duct, and so on from the first inner tube 50 near the outer tube 30.

### [Reference Signs List]

- 10, 10a, 10b: catheter
- 10c: balloon catheter
- 20: connector
- 30: outer tube
- 40, 40a, 40b, 40c: protective film
- 50: first inner tube
- 52: first front-end opening
- 54: first insertion opening
- 60: second inner tube
- 62: second front-end opening
- 64: second insertion opening
- 70: reinforcing member
- 80: balloon
- 82: supply port
- 84: inflation lumen
- 100: welded part
- 110, 110a, 110b, 110c: first welded part
- 120, 120a, 120b, 120c: second welded part
- 130, 130a, 130b, 130c: third welded part

## Claims

1. A catheter (10, 10a, 10b) comprising:
an outer tube (30); and
a first inner tube (50) made of a thermoplastic resin, that is inserted into the outer tube (30) and is welded to the outer tube (30) in a first welded part (110, 110a, 110b, 110c) ; **characterized in that**
a protective film (40, 40a, 40b, 40c) that is interposed between the outer tube (30) and the first inner tube (50) and is provided at least in a part of the first welded part (110, 110a, 110b, 110c), wherein
the protective film (40, 40a, 40b, 40c) is made of a resin having higher heat resistance than the resin of the first inner tube (50).

2. The catheter (10, 10a, 10b) according to claim 1, wherein the first inner tube (50) extends along the length of the catheter (10, 10a, 10b).

3. The catheter (10b) according to claim 1 or 2, wherein the protective film (40b) interposed between the first inner tube (50) and the outer tube (30) has an axial length (L2) that is equal to or longer than an axial length (L1) of the first welded part (110c).

4. The catheter (10, 10a, 10b, 10c) according to any one of claims 1 to 3, further comprising a second inner tube (60) made of a thermoplastic resin, that is inserted into the outer tube (30) and is welded to the first inner tube (50) in a second welded part (120, 120a, 120b, 120c).

5. The catheter (10, 10a, 10b, 10c) according to claim 4, wherein the second inner tube (60) extends from a midpoint to a front end of the catheter (10).

6. The catheter (10a) according to claim 4 or 5, wherein a protective film (40a) made of a resin having higher heat resistance than the resin of the first inner tube (50), is interposed between the first inner tube (50) and the second inner tube (60) and is provided at least in a part of the second welded part (120).

7. The catheter (10a) according to claim 6, wherein the protective film (40a) interposed between the first inner tube (50) and the second inner tube (60) is provided separately from the protective film (40) interposed between the first inner tube (50) and the outer tube (30).

8. The catheter (10b) according to claim 6, wherein the protective film (40b) interposed between the first inner tube (50) and the second inner tube (60) is formed by the protective film (40b) interposed between the first inner tube (50) and the outer tube (30) covering the outer periphery of the first inner tube (50).

9. The catheter (10b) according to any one of claims 6 to 8, wherein the protective film (40b) interposed between the first inner tube (50) and the outer tube (30) and the protective film (40a, 40b) interposed between the first inner tube (50) and the second inner tube (60) have an axial length (L2) that is equal to or longer than an axial length (L1) of the first welded part (110b) and the second welded part (120b).

10. The catheter (10b, 10c) according to any one of claims 4 to 9, wherein the second inner tube (60) is welded to the outer tube (30) in a third welded part (130b, 130c).

11. The catheter (10c) according to claim 10, wherein a protective film (40c) made of a resin having higher heat resistance than the resin of the second inner tube (60) is interposed between the second inner tube (60) and the outer tube (30).

12. The catheter (10c) according to claim 11, wherein the protective film (40c) interposed between the second inner tube (60) and the outer tube (30) covers the outer periphery of the second inner tube (60).

13. A balloon catheter (10c) comprising the catheter (10, 10a, 10b) according to any one of claims 1 to 12, and a balloon (80) welded to a front end of the outer tube.
